(19) Europäisches Patentamt
European Patent Office
Office européen des brevets

(11) Numéro de publication: **0 337 839 B1**

(12) # FASCICULE DE BREVET EUROPEEN

(45) Date de publication du fascicule du brevet: **19.09.90**

(51) Int. Cl.[5]: **C07C 319/24**, C07C 321/14

(21) Numéro de dépôt: **89400844.0**

(22) Date de dépôt: **24.03.89**

(54) **Procédé de fabrication des dialkyldisulfures inférieurs.**

(30) Priorité: **14.04.88 FR 8804964**

(43) Date de publication de la demande: **18.10.89 Bulletin 89/42**

(45) Mention de la délivrance du brevet: **19.09.90 Bulletin 90/38**

(84) Etats contractants désignés: **BE DE ES GB IT NL**

(56) Documents cités:
**EP-A-00 259 44**
**FR-A- 2 607 496**

(73) Titulaire: **SOCIETE NATIONALE ELF AQUITAINE (PRODUCTION), Tour Elf 2, Place de la Coupole La Défense 6, F-92400 Courbevoie(FR)**

(72) Inventeur: **Gongora, Henri, 5 Parc Résidence Chemin Salié, Lons F-64050 Billere(FR)**
Inventeur: **Darche, Yves, 15 Lotissement Bourdette, F-64300 Orthez(FR)**

(74) Mandataire: **Leboulenger, Jean et al, ATOCHEM Département Propriété Industrielle Cédex 42- La Défense 10, F-92091 Paris la Défense(FR)**

**EP 0 337 839 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne le domaine des disulfures organiques et a plus particulièrement pour objet la fabrication des dialkyldisulfures inférieurs R-SS-R où R désigne une radical alkyle contenant de 1 à 3 atomes de carbone.

Une importante voie d'accès à ces disulfures consiste en l'oxydation d'un alkylmercaptan par le soufre en présence d'un catalyseur selon le schéma réactionnel suivant

$$2\ R\text{-}SH + S \underset{}{\overset{\text{catalyseur}}{\rightleftharpoons}} R\text{-}SS\text{-}R + H_2S$$

Dans ce procédé où différentes réactions secondaires sont possibles, notamment :

$R\text{-}SS\text{-}R + S \rightleftharpoons R\text{-}SSS\text{-}R$

$2\ R\text{-}SS\text{-}R + H_2S \rightleftharpoons R\text{-}SSS\text{-}R + 2\ R\text{-}SH$

la formation du dialkyldisulfure est favorisée par un excès d'alkylmercaptan et l'élimination de l'hydrogène sulfuré.

Comme catalyseur on emploie le plus souvent des amines aliphatiques, en particulier la triéthylamine (brevets US 2 237 625, FR 1 358 398, US 3 299 146 et FR 2 130 985). Cette dernière présente l'inconvénient de se retrouver partiellement comme impureté dans le disulfure final et de lui conférer une coloration indésirable. D'autre part, pour le prétraitement des catalyseurs d'hydrocracking, certains utilisateurs de disulfures préfèrent disposer de produits sans azote.

Il a maintenant été trouvé que sans affecter la productivité de l'installation de production, on peut obtenir un dialkyldisulfure d'excellente qualité, exempt d'impuretés aminées et très faiblement coloré, en utilisant comme catalyseur la combinaison d'un alkylmercaptan supérieur R'-SH avec un oxyde d'alcène et une base alcaline. D'autre part, dans une installation de production continue de dialkyldisulfure, la combinaison catalytique selon l'invention présente, par rapport aux trialkylamines de la technique antérieure, l'avantage supplémentaire de pouvoir être récupérée et recyclée au réacteur de synthèse.

L'invention a donc pour objet un procédé de fabrication des dialkyldisulfures inférieurs R-SS-R où R désigne un radical alkyle contenant de 1 à 3 atomes de carbone, par oxydation de l'alkylmercaptan R-SH correspondant par le soufre en présence d'un catalyseur, caractérisé en ce que le catalyseur utilisé est une combinaison d'un alkylmercaptan supérieur R'-SH avec un oxyde d'alcène et une base alcaline, cette combinaison pouvant être représentée par la formule brute $R'\text{-}SH.x(C_nH_{2n}O).yMOH$

dans laquelle R' est un radical alkyle, linéaire ou ramifié, contenant de 6 à 18 atomes de carbone, n est un nombre allant de 2 à 4, x un nombre allant de 1 à 20, y un nombre allant de 0,01 à 1 et M désigne un atome de métal alcalin, de préférence Na ou K.

Les combinaisons préférées sont celles dans lesquelles R' est un radical alkyle tertiaire en $C_8$ à $C_{16}$ (par exemple tert-nonyle ou tert-dodécyle), le nombre x de moles d'oxyde d'alcène par mole de mercaptan R'-SE est compris entre 4 et 12, et le nombre y de moles de base alcaline est compris entre 0,1 et 0,5. Comme oxyde d'alcène, on utilise de préférence l'oxyde d' éthylène plus économique, mais il peut éventuellement être remplacé par ou mélangé à l'oxyde de propylène ou de butylène.

Un mode de préparation de la combinaison catalytique selon l'invention consiste à mélanger d'abord 1 mole de mercaptan R'SH avec $\alpha y$ moles ($\alpha = 1$ à 1,5) de base alcaline MOH, de préférence en homogénéisant ce mélange à chaud; cette opération peut s'effectuer notamment en quelques heures entre 50 et 100°C, en particulier en 4 à 6 heures entre 75 et 85°C. Dans le liquide ainsi obtenu, on injecte ensuite l'oxyde d'alcène $C_nH_{2n}O$ de façon à fixer la proportion x désirée ; cette injection s'effectue de préférence sous 0,2 à 4 bars et à une température de 80 à 120°C selon la nature du mercaptan R'SS utilisé, et la fixation de l'oxyde d'alcène dure en général de 1 à 10 heures. Après ces opérations, il est recommandé d'éliminer l'excès d'oxyde d'alcène par dégazage du mélange réactionnel au moyen d'un gaz inerte tel que l'azote, puis de filtrer le liquide obtenu avant son utilisation comme catalyseur dans le procédé selon l'invention.

La quantité de catalyseur à mettre en oeuvre pour la réaction d'oxydation conduisant aux disulfures RSSR peut aller de 0,2 à 5 % par rapport au poids de soufre utilisé et est, de préférence, comprise entre 1,5 et 4 %.

Comme dans les procédés connus, le rapport molaire mercaptan RSH/soufre doit être au moins égal à 2 et est, de préférence, compris entre 3 et 5.

La réaction d'oxydation peut être effectuée à une température allant de 25 à 125°C sous une pression effective allant de 1 à 5 bars, de préférence comprise entre 3 et 4 bars. La température optimale dépend du mercaptan RSH mis en oeuvre; elle est de 50-60°C pour le méthylmercaptan, de 60-70°C pour l'éthylmercaptan et de 70-80°C pour le propylmercaptan.

Bien que la combinaison catalytique selon l'invention puisse être utilisée dans un procédé discontinu de production des dialkyldisulfures R-SS-R, elle est plus particulièrement destinée à la production de ces dialkyldisulfures dans une installation à fonctionnement continu.

La figure annexée est une représentation schématique d'une telle installation qui comprend notamment un réacteur 1, une colonne de dégazage 2 et une colonne de distillation 3.

Les réactifs soufre liquide, mercaptan RSH (liqùide ou gazeux) et catalyseur (ou fonds recyclés provenant du bas de la colonne 3) sont introduits en continu respectivement par les conduites 4, 5 et 6 dans le réacteur 1 équipé des dispositifs usuels de régulation en température, pression, niveau et d'une boucle de recirculation externe 7 permettant d'assurer les transferts massiques et thermiques. Le temps de séjour des réactifs dans ce réacteur peut aller de 20 à 180 minutes. Il est de préférence compris entre 30 et 90 minutes et est avantageusement d'environ une heure. Les effluents gazeux sont éliminés en tête du réacteur par la conduite 8 vers le circuit torche et le brut réactionnel liquide, soutiré en bas du réacteur, est amené par la canalisation 9 dans la colonne de dégazage 2.

Cette colonne à garnissage, munie d'une circulation d'eau chaude et d'une arrivée de gaz inerte tel que le méthane ou l'azote, permet de séparer la majeure partie de l'hydrogène sulfuré co-produit et de l'alkylmercaptan RSH excédentaire. Ce dernier est ensuite amené par la conduite 10 dans un échangeur-dévésiculeur où il est condensé avant recyclage au réacteur 1 soit directement, soit via un ballon de stockage. On récupère en fond de colonne 2 un produit brut constitué par le dialkyldisulfure RSSR désiré, des polysulfures correspondants (principalement du dialkyltrisulfure), le catalyseur et un peu d' alkylmercaptan.

Ce produit brut est amené par la canalisation 11 dans la colonne 3 où il est distillé sous pression réduite. On récupère en tête par la canalisation 12 le dialkyldisulfure désiré, tandis que les fonds constitués principalement par un mélange de dialkyldisulfure, dialkyltrisulfure et catalyseur sont recyclés au réacteur 1 par les canalisations 13 et 6.

L'exemple suivant illustre l'invention sans la limiter.

EXEMPLE

a) Préparation du catalyseur

Dans un réacteur de 150 litres en acier inoxydable, on introduit 68 kg (336,6 moles) de tert-dodécylmercaptan $C_{12}H_{25}SH$ avec 6,3 kg (157,5 moles) de NaOH anhydre, puis on assure une bonne dispersion et homogénéisation du mélange par chauffage à 80°C pendant 5 heures. On injecte ensuite de l'oxyde d'éthylène dans le réacteur avec un débit continu de 7 kg/h pendant 8 heures; la pression relative dans le réacteur passe de 0,2 bar au début à 4 bars à la fin de l'introduction. Le mélange réactionnel est ensuite chauffé à 100-110°C pendant 2 heures, puis décomprimé et dégazé au moyen d'un courant d'azote pour éliminer tout l'oxyde d'éthylène excédentaire dissous. Le liquide obtenu est alors soumis à une filtration pour arrêter les particules solides jusqu'à 5 µm.

La combinaison catalytique ainsi obtenue présente la composition molaire suivante :
$C_{12}H_{25}SH.6(CH_2CH_2O).0{,}35NaOH$
soit en poids :

$C_{12}H_{25}SH$ ....42 %
$CH_2CH_2O$ ....55 %
NaOH ....3 %

Le point de trouble de ce catalyseur est à environ 62°C.

b) Synthèse continue du diméthyldisulfure (DMDS)

On opère dans une installation du type décrit ci-dessus. Le réacteur 1 est alimenté en continu avec 10 kg/h de soufre liquide, 55 kg/h de méthylmercaptan liquide et 0,35 kg/h du catalyseur obtenu au paragraphe a). Lorsque l'équilibre de marche est atteint (après environ 12 heures), on remplace cette alimentation de 0,35 kg/h de catalyseur par le recyclage, à raison de 7 kg/h, du produit issu du fond de la colonne de distillation 3 qui contient 0,35 kg de catalyseur, 3,64 kg de DMDS et 3,01 kg de polysulfures, principalement du diméthyltrisulfure (DMTS).

La réaction s'effectue vers 55-58°C sous une pression de 3,5 bars effectifs, le temps de séjour moyen des réactifs dans le réacteur étant d'environ une heure. Le brut réactionnel liquide sortant du réacteur est amené dans la colonne 2 où il est soumis à un dégazage vers 65-70°C à l'aide d'un courant de 6 $Nm^3/h$ de gaz commercial (méthane).

On récupère en bas de la colonne 2, 37,26 kg/h d'un liquide contenant en poids 85,70 % de DMDS, 12,23 % de DMTS, 1,13 % de méthylmercaptan et 0,94 % de catalyseur. Ce liquide est envoyé dans la colonne de distillation 3 où il est fractionné dans les conditions suivantes :

débit d'alimentation: environ 37 kg/h
" " de distillation : environ 28,1 kg/h
" " de fond : environ 8,5 kg/h
pression partielle en tête : 13,33 kPa

température tête : 56°C
température fond : 90-100°C

On obtient en tête de colonne 28,1 kg/h de DMDS ayant une pureté de 99 %, soit un rendement de 95 % par rapport au soufre engagé. Le DMDS ainsi produit est très faiblement coloré; il est exempt d'impuretés aminées, de soude et d'oxygène et sa teneur en $CS_2$ résiduel est inférieure à 500 ppm.

On récupère en fond de colonne 8,3 kg/h d'un mélange contenant en poids environ 43,85 % de DMDS, 29,05 % de DMTS, 4,2 % de catalyseur et 22,9 % de polysulfures de rang supérieur. Après filtration, on obtient 7 kg/h d'un produit contenant environ 5% de catalyseur, 52 % de DMDS et 34,4 % de DMTS. Une fois l'équilibre de marche atteint, ce produit est introduit en remplacement du catalyseur frais.

## Revendications

1. Procédé de fabrication d'un dialkyldisulfure inférieur du type R-SS-R où R désigne un radical alkyle contenant de 1 à 3 atomes de carbone, par oxydation de l'alkylmercaptan RSH correspondant par le soufre en présence d'un catalyseur, caractérisé en ce que le catalyseur employé est une combinaison d'un alkylmercaptan supérieur R'-SH avec un oxyde d'alcène $C_nH_{2n}O$ et une base alcaline MOH, cette combinaison pouvant être représentée par la formule brute :
$R'\text{-}SH.x(C_nH_{2n}O).yMOH$
dans laquelle R' est un radical alkyle, linéaire ou ramifié, contenant de 6 à 18 atomes de carbone, n est un nombre allant de 2 à 4, x un nombre allant de 1 à 20, y un nombre allant de 0,01 à 1 et M désigne un atome de métal alcalin.

2. Procédé selon la revendication 1, dans lequel R' est un radical alkyle tertiaire en $C_8$ à $C_{16}$, de préférence tert-nonyle ou tert-dodécyle.

3. Procédé selon la revendication 1 ou 2, dans lequel n est égal à 2, x est compris entre 4 et 12 et y est compris entre 0,1 et 0,5.

4. Procédé selon l'une des revendications 1 à 3, dans lequel le catalyseur est utilisé en une quantité de 0,2 à 5 % par rapport au poids de soufre introduit, le rapport molaire mercaptan RSH/soufre étant au moins égal à 2 et, de préférence, compris entre 3 et 5.

5. Procédé selon l'une des revendications 1 à 4, dans lequel la réaction est effectuée à une température allant de 25 à 125°C, de préférence comprise entre 50 et 80°C, et sous une pression effective allant de 1 à 5 bars, de préférence comprise entre 3 et 4 bars.

6. Procédé selon l'une des revendications 1 à 5, dans lequel on opère en continu, le temps de séjour dans le réacteur allant de 20 à 180 minutes et étant, de préférence, compris entre 30 et 90 minutes.

7. Procédé selon l'une des revendications 1 à 6, dans lequel l'alkylmercaptan RSH est le méthylmercaptan.

## Claims

1. Process for the production of a lower dialkyl disulphide of the type R-SS-R, where R denotes an alkyl radical containing from 1 to 3 carbon atoms, by oxidation of the corresponding alkyl mercaptan RSH by sulphur in the presence of a catalyst, characterized in that the catalyst employed is a combination of a higher alkyl mercaptan R'-SH with an alkene oxide $C_nH_{2n}O$ and an alkali metal base MOH, it being possible to represent this combination by the empirical formula:
$R'\text{-}SH.x(C_nH_{2n}O).yMOH$
in which R, is a straight-chain or branched alkyl radical containing from 6 to 18 carbon atoms, n is a number ranging from 2 to 4, x is a number ranging from 1 to 20, y is a number ranging from 0.01 to 1 and M denotes an atom of alkali metal.

2. Process according to Claim 1, in which R' is a $C_8$ to $C_{16}$ tertiary alkyl radical, preferably tert-nonyl or tert-dodecyl.

3. Process according to Claim 1 or 2, in which n is 2, x is between 4 and 12 and y is between 0.1 and 0.5.

4. Process according to one of Claims 1 to 3, in which the catalyst is used in an amount of 0.2 to 5% relative to the weight of sulphur introduced, the mercaptan RSH/sulphur molar ratio being at least 2 and preferably between 3 and 5.

5. Process according to one of Claims 1 to 4, in which the reaction is carried out at a temperature ranging from 25 to 125°C, preferably between 50 and 80°C, and under an effective pressure ranging from 1 to 5 bars, preferably between 3 and 4 bars.

6. Process according to one of Claims 1 to 5, in which the reaction is carried out continuously, the residence time in the reactor ranging from 20 to 180 minutes and preferably being between 30 and 90 minutes.

7. Process according to one of Claims 1 to 6, in which the alkyl mercaptan RSH is methyl mercaptan.

## Patentansprüche

1. Verfahren zur Herstellung eines niederen Dialkyldisulfids des Typs R–SS–R, in dem R einen 1 bis 3 Kohlenstoffatome enthaltenden Alkylrest bezeichnet, durch Oxidation des entsprechenden Alkylmercaptans RSH durch Schwefel in Gegenwart eines Katalysators, dadurch gekennzeichnet, daß der eingesetzte Katalysator eine Kombination aus einem höheren Alkylmercaptan R'-SH, einem Alkenoxid $C_nH_{2n}O$ und einer Alkalibase MOH ist, diese Kombination kann durch die Brutto-Formel wiedergegeben werden:
$R'\text{-}SH.x(C_nH_{2n}O).yMOH$
in der R' ein geradkettiger oder verzweigter, 6 bis 18 Kohlenstoffatome enthaltender Alkylrest ist, n ist eine Zahl von 2 bis 4, x eine Zahl von 1 bis 20, y eine Zahl von 0,01 bis 1 ist und M ein Alkalimetallatom bezeichnet.

2. Verfahren nach Anspruch 1, in dem R' ein tertiärer $C_8$ bis $C_{16}$-Alkylrest ist, vorzugsweise tert.-Nonyl oder tert.-Dodecyl ist.

3. Verfahren nach Anspruch 1 oder 2, in dem n gleich 2 ist, x zwischen 4 und 12 und y zwischen 0,1 und 0,5 liegt.

4.Verfahren nach einem der Ansprüche 1 bis 3, in dem der Katalysator in einer Menge von 0,2 bis 5%, bezogen auf das Gewicht des eingeführten Schwefels, verwendet wird, das Molverhältnis Mercaptan RSH/ Schwefel ist mindestens gleich 2 und vorzugsweise zwischen 3 und 5.

5. Verfahren nach einem der Ansprüche 1 bis 4, in dem die Reaktion bei einer Temperatur von 25 bis 125°C, vorzugsweise zwischen 50 und 80°C und unter einem Reaktionsdruck von 1 bis 5 bar, vorzugsweise zwischen 3 und 4 bar, durchgeführt wird.

6. Verfahren nach einem der Ansprüche 1 bis 5, in dem man kontinuierlich arbeitet, die Verweilzeit im Reaktor 20 bis 180 Minuten und vorzugsweise zwischen 30 und 90 Minuten beträgt.

7. Verfahren nach einem der Ansprüche 1 bis 6, in dem das Alkylmercaptan RSH Methylmercaptan ist.

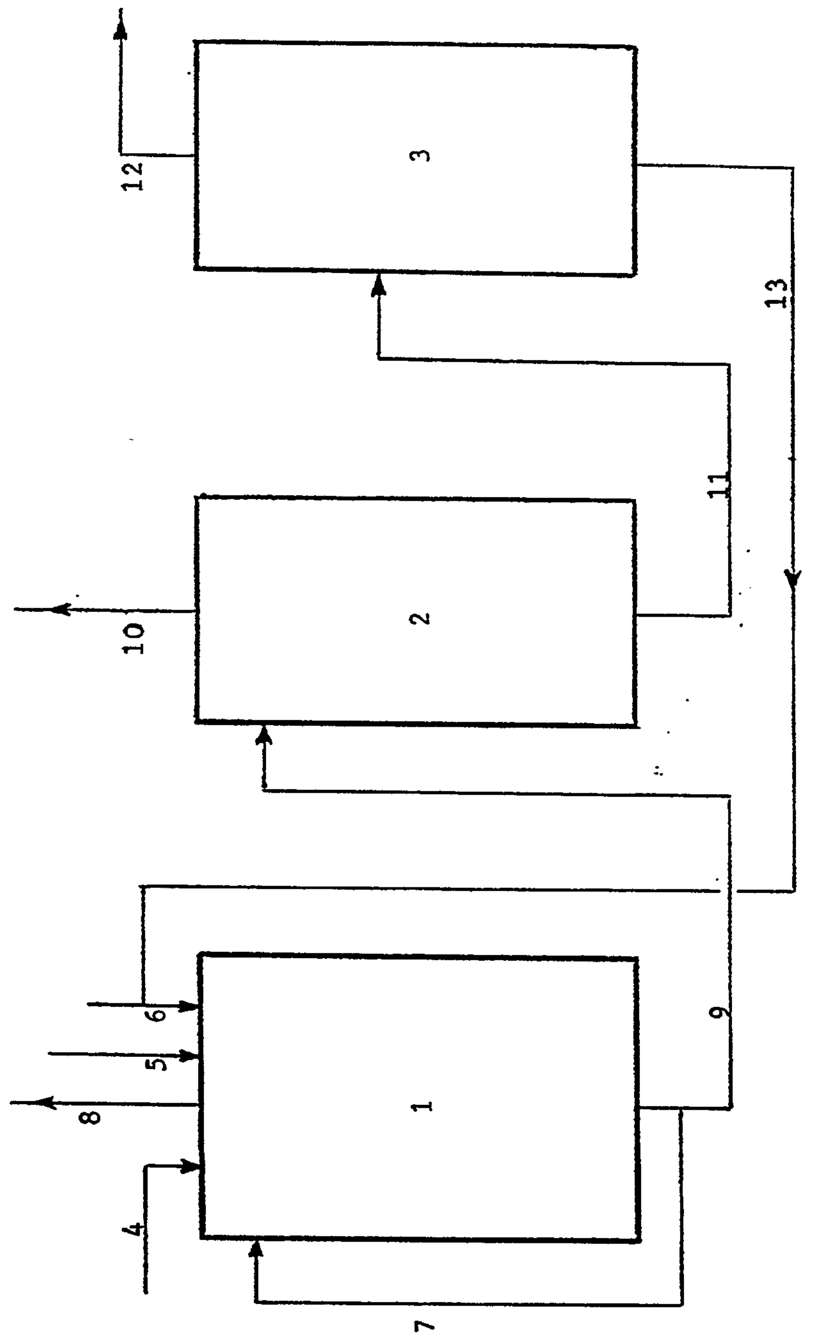

1
2
3
4
5
6
7
8
9
10
11
12
13